# EUROPEAN PATENT APPLICATION

(11) **EP 4 417 619 A1**
(43) Date of publication of application: **21.08.2024**
(21) Application number: 21960763.7
(22) Date of filing: 12.10.2021
(51) Int. Cl.: C07K 14/47, C07K 19/00, C12N 15/62, C12N 15/63, C12N 5/10, A61K 47/68

(54) **NUCLEOTIDE SEQUENCE ENCODING A FUSION PROTEIN**

(71) Applicant: "Palmira Biopharma" Limited Liability Company, Moscow, 121205 (RU)
(72) Inventor: AKSENOVA, Anna Yurievna, St. Petersburg, 198516 (RU); VOLKOV, Kirill Vladimirovich, St. Petersburg, 198516 (RU); VORONINA, Ekaterina Vladimirovna, Moscow, 117519 (RU); MARYGIN, Roman Andreevich, g. Zarechny, 442962 (RU); ASKRETKOV, Alexander Dmitrievich, r.p. Novodrozhzhino, 142720 (RU); ZAVARZIN, Alexei Alekseevich, St. Petersburg, 197022 (RU); LUKYANOV, Dmitry Valerievich, St. Petersburg, 195257 (RU); SHEVCHENKO, Konstantin Georgiyevich, St. Petersburg, 196191 (RU); SAIFITDINOVA, Alsu Faritovna, St. Petersburg, Peterhof, 198504 (RU); SEMENIKHIN, Vyacheslav Alekseevich, Moscow, 119602 (RU)
(74) Representative: Jeck, Jonathan
(86) International application number: PCT/RU2021/000434
(87) International publication number: WO 2023/063842

(57) **Abstract**

The present invention describes nucleic acid molecules and new recombinant polypeptides encoded by them, the polypeptides being capable of blocking DII4-Notch signaling pathways and consisting of a soluble extracellular fragment of human DII4 and the constant part of the heavy chain of human IgG4. The plasmid structure that allows expressing the fusion protein encoded by the specified nucleic acids was constructed using genetic engineering methods. The developed hybrid protein constructs obtained as a result of expression of these nucleic acid constructs have a low molecular weight, which increases the efficiency of the production of these molecules in mammalian cells and improves their properties related to the rate and extent of penetration into tissues for the treatment of ischemic conditions, cancers, and other diseases associated with the pathological activity of Notch receptors or when modulation of the function of the DII4-Notch signaling pathway is appropriate.

## Description

### Technical field

The invention is in the field of biomolecular pharmacology, biotechnology and genetic engineering and concerns nucleic acid molecules, new soluble proteins encoded by them, the proteins being functional antagonists of human DII4 (Delta-like ligand 4) capable of blocking DII4-Notch signaling pathways, and a method of their production.

### Background art

DII4 is a transmembrane protein, a ligand for Notch receptors, including Notch1 and Notch4. By activating these receptors, DII4 participates in angiogenesis, down-regulates the proliferation and migration of endothelial cells and sprouting angiogenesis. Numerous experiments have shown that blockade of the interaction of DII4 with its receptors leads to stimulation of productive angiogenesis in ischemic tissues, improving their supply of oxygen and nutrients. Blockade of the interaction of DII4 with its receptors also has an anti-inflammatory effect in the vascular endothelium, which is due to inhibition of the expression of angiotensinogen, and a vasodilating effect (Lobov et al. The DII4/Notch pathway controls postangiogenic blood vessel remodel-ing and regression by modulating vasoconstriction and blood flow // Blood, 2011, Vol 117, Number 24:6728-37 doi: 10.1182/blood-2010-08-302067). In addition, the anticancer effect of inhibitors of the interaction of DII4 with its receptors is known, and it can be achieved through several mechanisms, including inhibition of the expression of the cellular oncogene *MYC,* stimulation of non-productive angiogenesis in the tumor interfering with its perfusion, etc.

One of the approaches to blocking the interaction of DII4 with its receptors is the development of competitive antagonists that bind to the Notch receptors without activating them.

The literature describes an antagonist of the Delta-like ligand DII4, which is a fusion DII4-Fc polypeptide that can participate in the regulation of angiogenesis. In particular, intraperitoneal administration of DII4-Fc in mice with ischemic extremities at a concentration of 2.5 mg/kg was found to stimulate the growth and maturation of new vessels and thus improve the blood supply to the extremities (Liu R. et al. Inhibition of Notch signaling by DII4-Fc promotes reperfusion of acutely ischemic tissues // Biochemical and Biophysical Research Communications, 2012, Vol. 418, No 1, p. 173-179).

The use of the fusion DII4-Fc polypeptide as a modulator of Notch signaling, vascular growth stimulation, blood pressure regulation, and inflammation markers is also described in an article by Lobov et al., Blood, 2011.

A method of treatment of ischemic vascular diseases of the eye with DII4 antagonists is known, as described in patent EP2054082B1. In this method, a Delta ligand 4 (DII4) antagonist, which includes an extracellular DII4 domain optionally bound to a multimerized component, is used to prevent or reduce blood vessel loss and/or promote productive angiogenesis in a patient with ischemic injury or vascular insufficiency. In specific embodiments, the DII4 antagonist approximately contains amino acids 27-172, 27-217, 218-400, 218-360, 218-322, or 218-282 of the amino acid sequence of the DII4 protein (SEQ ID NO: 1).

In the angiogenesis activation method described in US2007213266A1, patients are administered an effective amount of the therapeutic polypeptide, which is the extracellular part of the DII4 protein, in one of the embodiments of the described method, i.e., the DSL domain of the DII4 protein.

However, the implementation of the approaches described above has a number of limitations: the affinity of binding of known therapeutic polypeptides to the Notch receptor is not high enough, and the large size of therapeutic polypeptides prevents their proper penetration into tissues and requires a high dose of the drug to be administered to the patient, which, in turn, can lead to sensitization of the patient and other adverse effects. Also, the use of DII4-Fc molecules with a full-size extracellular DII4 domain in the composition can lead to activation of Notch signal transmission. To date, despite the promising potential, there are still no clinically approved drugs based on the fusion DII4-Fc peptide.

The proposed invention has a number of improved properties in comparison with analogs, and therefore expands the range of available therapeutic candidates working by blocking the interaction of DII4 with Notch receptors.

### Character of the invention

The purpose of this invention is to expand the technical armamentarium for the treatment of diseases of the cardiovascular system, ischemic disorders, vascular inflammation, and cancers, to stimulate therapeutic angiogenesis. In particular, the objective was to develop new highly effective DII4 antagonist polypeptides with high affinity for the Notch receptor, relatively small in size and not activating the transmission of the Notch signal during the interaction; the objective also involved the development of nucleotide sequences encoding such polypeptides and being the expression basis for their production.

This task is solved by creating a fusion hDII4-hFc protein antagonizing DII4 and containing a fragment of the soluble extracellular part of the human DII4 protein, including the following functional subunits: the DSL domain of the DII4 protein (173-217 SEQ ID NO: 1), at least five domains of the EGF-like repeats of the DII4 protein represented by fragments 218-251, 252-282, 284-322, 324-360, 362-400 of the SEQ ID NO: 1 sequence; as well as the constant part of the heavy chain (Fc region) of human IgG4. The Fc region of human IgG4 is attached to the C-terminal fragment of the DII4 protein, directly or via a linker sequence.

In some embodiments of the invention, the fusion protein additionally includes 1-3 domains of the EGF-like repeats of the DII4 protein represented by fragments 402-438, 440-476 and 480-518 of the SEQ ID NO: 1 sequence. In other words, the fusion protein additionally includes at least one of domains 6-8 of the EGF-like repeats of the DII4 protein located at the C-terminus of the DII4 protein fragment, and these domains 6-8 of the EGF-like repeats may be located in any order.

In some embodiments of the invention, the Fc region of human IgG4 is connected to the DII4 protein fragment without a linker sequence. In some other embodiments of the invention, the Fc region of human IgG4 is connected to the DII4 protein fragment via a linker peptide. In some particular embodiments of the invention, the linker peptide is the dipeptide GS.

In some embodiments of the invention, the fragment of the soluble extracellular part of the human DII4 protein is included in the structure of the fusion protein in the form of a SEQ ID NO: 6 sequence.

In some embodiments of the invention, the constant part of the heavy chain (Fc region) of human IgG4 has the SEQ ID NO: 7 sequence.

In some embodiments of the invention, the fusion protein additionally includes a signal sequence located at the N-terminus of the fusion protein.

In some particular embodiments of the invention, the fusion protein has an amino acid sequence of SEQ ID NO: 2.

In some other particular embodiments of the invention, the fusion protein has an amino acid sequence of SEQ ID NO: 4.

The task is also solved by creating an isolated nucleic acid molecule encoding such a fusion protein.

In some particular embodiments of the invention, the nucleic acid molecule has a sequence of SEQ ID NO: 3.

In some other particular embodiments of the invention, the nucleic acid molecule has a sequence of SEQ ID NO: 5.

This task is also solved by creating an expression vector carrying the nucleotide sequence corresponding to the sequence of the isolated nucleic acid molecule encoding such a fusion protein, under the control of regulatory elements necessary for the expression of this nucleotide sequence in the host cell; and by creating a host cell incorporating the expression vector described in this application, with the host cell ensuring the effective production of such a fusion protein using the aforementioned nucleic acid molecule. In some embodiments of the invention, the cells used are mammalian cells (in some particular embodiments of the invention, Chinese hamster ovary (CHO) cells are used).

The solution of this task is also achieved by the use of the above-mentioned fusion protein for the treatment of a wide range of ischemic conditions (in particular, lower limb ischemia), cardiovascular diseases, cancers, and the treatment of vascular complications of infectious diseases, including COVID-19.

Also, the solution of this task is achieved by using the method for the treatment of a wide range of ischemic conditions (in particular, lower limb ischemia), cardiovascular diseases, cancers, and the treatment of vascular complications of infectious diseases, including COVID-19, which includes the administration to the patient of the fusion protein described in the invention. According to the invention, such a protein is administered to the patient in a therapeutically effective amount, preferably as part of a pharmaceutical composition.

The implementation of the invention achieves the following technical effects:
- new variants of the therapeutic agent based on the fusion (hybrid) hDII4-hFc protein have been created, which contain the functional domains of the DSL and EGF-like repeats (five to eight) of the DII4 protein fused with the constant part (Fc domain) of human immunoglobulin IgG4 and capable of selectively blocking Notch signaling, and nucleotide sequences encoding such fusion proteins have been developed and serve as the expression basis for their production;
- the developed hybrid constructs have only minimal effector function of antibody-dependent cellular cytotoxicity and do not exhibit complement-dependent cytotoxicity, which reduces the possible risk of inflammatory reactions and sensitization with the use of the therapeutic agent based on the fusion (hybrid) hDII4-hFc protein;
- the developed hybrid constructs include the minimum fragment of human DII4 protein (the DSL domain and, at a minimum, the first 5 domains of the EGF-like repeats), which is required and sufficient for interaction with Notch receptors;
- the developed hybrid constructs do not include the MNNL domain of human DII4 protein involved in the activation of Notch signal transmission;
- the developed hybrid constructs have a relatively low molecular weight, which increases the efficiency of the production of these molecules in mammalian cells and improves their properties related to the rate and extent of penetration into tissues;
- the developed hybrid constructs, due to the absence of the MNNL domain in the construct, have decreased aggregation capacity and improved solubility;
- binding of the developed hybrid constructs to Notch receptors does not lead to their activation, due to the absence of the MNNL domain in the design; as a result, such binding leads to effective blockade of Notch signaling.

### Brief description of the figures

Figure 1. Scheme of the expression vector containing the nucleotide sequence encoding the fusion protein with an amino acid sequence of SEQ ID NO: 2.
Figure 2. Scheme of the expression vector containing the nucleotide sequence encoding the fusion protein with an amino acid sequence of SEQ ID NO: 4.

### Definitions and terms

The following terms and definitions apply in this document, unless otherwise specified. References to the procedures used in the description of this invention refer to well-known methods, including modifications of these methods and their replacement with equivalent methods known to those skilled in the art.

In the documents of this invention, the terms "includes", "including", etc., and "contains", "containing", etc. are interpreted to mean "includes, among other things" (or "contains, among other things"). These terms are not intended to be interpreted as "consists only of".

The terms "polypeptide", "protein", and "peptide" are used interchangeably in this document, and all refer to a polymer made of amino acid residues. These terms may also be used interchangeably in this description when referring to the end product resulting from the expression of a nucleic acid sequence in a host cell.

A "fusion protein" ("hybrid protein"," recombinant protein"," fusion polypeptide"," recombinant polypeptide"," hybrid construct") is a construct containing of two or more parts of a polypeptide nature, covalently bound, formed as a result of the expression of a recombinant DNA molecule, in which the coding regions are connected to each other in one reading frame, respectively, of two or more different genes or their fragments.

The term "isolated" has its conventional meaning known to the average person skilled in the art; when used in relation to an isolated nucleic acid or isolated polypeptide, it is used without limitation to refer to a nucleic acid or polypeptide that, when handled by humans, exists separately from its native environment and is therefore not a product of nature. An isolated nucleic acid or polypeptide may exist in a purified form or may exist in a non-native environment, such as a host cell.

The term "synonymous substitution" refers to a nucleotide sequence having a nucleotide sequence that may differ from the reference nucleic acid sequence in one or more substitutions that do not lead to a change in the amino acid sequence of the protein encoded by the nucleic acid molecule. Since the genetic code is "degenerate", that is, different triplets in the nucleotide sequence can encode the same amino acid, synonymous substitutions in the nucleotide sequence do not lead to a change in the amino acid sequence of the protein.

The term "decoy ligand" (or "trap") refers to hybrid proteins consisting of the extracellular ligand domain of the target molecule (receptor) and the Fc domain of the immunoglobulin. The extracellular domain of the ligand is responsible for binding the target, and the Fc domain dimerizes the hybrid protein. The latter is necessary to increase the binding efficiency and ensure greater stability of the high-molecular-weight complex. In the framework of this invention, the "decoy ligand" consists of certain fragments of the soluble extracellular fragment of the human DII4 protein (the DSL domain and, at a minimum, the first 5 domains of the EGF-like repeats) and the constant part of the heavy chain of human IgG4.

The term "treatment" means curing, slowing down, arresting, or reversing the progression of a disease or disorder. In this document, "treatment" also means the relief of symptoms associated with a disease or disorder.

"Therapeutically effective amount (therapeutic dose)" means the amount of a drug administered to a patient at which the expected therapeutic effect is most likely to occur. The exact amount required may vary from subject to subject depending on multiple factors, such as disease severity, age, body weight, general condition of the patient, combination treatment with other drugs, etc. According to the invention, the medicinal product is administered to a subject requiring treatment and/or prevention of a disease or condition at a dose sufficient to achieve a therapeutic effect. During the treatment and/or prophylaxis, either single or multiple daily doses may be used, usually in a course given over a period of time sufficient to achieve a therapeutic effect (from several days to a week, several weeks and up to months), and courses of the drug may be repeated. In particular, in moderate forms of a disease, the single dose, dosing frequency, and/or duration of administration of the medicinal product may be increased, according to the invention. Preferably, the fusion protein described in the invention is administered to the patient in a pharmaceutical composition that includes, in addition to the active ingredient, pharmaceutically acceptable carriers and/or fillers.

Unless otherwise specified, technical and scientific terms in this application have the standard meanings generally accepted in the scientific and technical literature.

### Detailed description of the invention

This invention proposes DNA constructs encoding fusion hDII4-hFc proteins (recombinant polypeptides), which are DII4 antagonists that block the Notch signaling pathway, but are relatively small in size compared to decoy ligands using the full-size ectodomain of human DII4 protein, which results in their improved properties in terms of the rate and extent of penetration into tissues, protein production in mammalian cells, and the absence of activation of the Notch signal transmission following binding.

In particular, the invention relates to nucleotide constructs encoding polypeptides containing the functional DSL domains and EGF-like repeats (five to eight) of the DII4 protein fused with the constant part (Fc domain) of human immunoglobulin IgG4, hDII4-hFc.

The natural sequence of human DII4 protein consists of 685 amino acids (AA) (SEQ ID NO: 1). The protein consists of a signal peptide (1-26 AA SE SEQ ID NO: 1), a transmembrane domain (530-550 AA SE SEQ ID NO: 1), a Delta/Serrate/Lag-2 domain (DSL, 173-217 AA SE SEQ ID NO: 1), and eight tandem repeats similar to epidermal growth factor (domains of the EGF-like repeats) (218-251, 252-282, 284-322, 324-360, 362-400, 402-438, 440-476, 480-518 AA SEQ ID NO: 1). The protein has an extracellular part (27-529 AA SE SEQ ID NO: 1) and a cytoplasmic part (551-685 AA SE SEQ ID NO: 1), while the N-terminus of the DII4 protein (27-172 AA SE SEQ ID NO: 1) forms a separate functional domain, MNNL. The state of the art includes studies of various authors who examined the structure and role of different domains of the DII4 protein in the interaction with Notch receptors and signaling (see, for example, Luca et al. Structural basis for Notch1 engagement of Delta-like 4 // Science, 2015, Vol 347, Issue 6224, pp. 847-853 doi: 10.1126/science.1261093; Hirano et al. Delta-like 1 and Delta-like 4 differently require their extracellular domains for triggering Notch signaling in mice // Elife 2020; 9: e50979. Published online 2020 Jan 16 doi: 10.7554/eLife.50979); however, at this time, not all the subtleties and specific features of Notch signaling have been fully elucidated.

Within the framework of this invention, in the process of development of new highly effective polypeptide drugs antagonizing DII4, it was assumed that the absence of the MNNL domain in the hybrid polypeptide would allow blocking Notch signaling. In accordance with this task, nucleotide sequences were created that encode therapeutic molecules capable of selectively blocking Notch signaling, which are hybrid polypeptides antagonizing DII4 (hDII4-hFc). These protein molecules include the functional DSL domains and EGF-like repeats (at least the first five domains) of the DII4 protein, fused with the constant part (Fc region) of human IgG4. Blockade of the Notch signal transmission during the interaction with the Notch receptor is achieved due to the absence in the therapeutic molecule of the MNNL domain, which participates in the signal transmission. Such a construct provides, on the one hand, the antagonistic properties of the molecule described in the invention and, on the other hand, a decreased size of the molecule. The size of the molecule according to the invention can also be further reduced by decreasing the number of EGF-like repeat domains (as shown by the experiments, the antagonistic properties of the molecule are preserved when 5 to 8 EGF domains are included). According to the invention, the functional domains of the DII4 protein can be fused with the constant part of the heavy chain of human IgG4 both directly (i.e., without a linker sequence) and via a linker sequence. The fusion hDII4-hFc protein described in the invention also does not necessarily contain a signal sequence, which may include any sequence known to those skilled in the management of the secretion of a polypeptide or protein from the cell, and may include natural or synthetic sequences. Normally, the signal sequence is located at the N-terminus of the fusion protein described in this invention.

In particular embodiment of the invention, recombinant polypeptides according to the invention include the DSL domain of DII4 and the EGF(1-5) domains of the DII4 protein or the DSL domain of DII4 and the EGF (1-8) domains of the DII4 protein, and are soluble recombinant decoy (trap) ligands antagonizing DII4. Such molecules inhibit the Notch signaling pathway by specifically binding to the Notch1 and Notch4 receptors. This does not activate the Notch pathway; instead, hDII4-hFc molecules prevent the binding of Notch receptors to endogenous full-length activating ligands. The Notch1 and Notch4 signaling pathways are involved in the control of blood vessel growth, and their inhibition by the action of hDII4-hFc leads to activation of the growth of new vessels (therapeutic angiogenesis) in ischemic tissues and improves wound healing. Specific inhibition of the Notch signaling pathways through the use of DII4 antagonists can be used to treat a wide range of cardiovascular diseases and cancers, as well as to treat vascular complications of infectious diseases, including COVID-19.

The invention allows to obtain polypeptides with high affinity for human Notch receptors, but with a reduced molecular weight compared to natural DII4 proteins, as well as compared to the DII4-Fc molecules described earlier. Reducing the size of recombinant polypeptides allows to increase the rate of their penetration into the body tissues, reduce the doses of the drug (pharmaceutical composition) containing such polypeptides that have to be administered to patients, and decrease the costs of synthesis of the described recombinant polypeptides.

Drugs based on the described recombinant polypeptides can be used for the treatment of ischemic diseases of various organs and tissues. Ischemia is decreased blood supply to an area of the body, organ or tissue due to low or no influx of arterial blood to it. Ischemia can be caused by various factors and occurs when the blood flow does not meet the needs of the organ. There are a number of therapeutic approaches to solving the problems of restoring blood flow and treating ischemic damage to various tissues, one of which is therapeutic angiogenesis, i.e., the drug-induced formation of new functioning vessels growing from existing ones.

The described DNA constructs allow producing hDII4-hFc molecules that have a low molecular weight and high bioavailability and can be used to stimulate therapeutic angiogenesis, treat a wide range of ischemic conditions (in particular, lower limb ischemia), cardiovascular diseases, cancers, treat vascular complications of infectious diseases, including COVID-19, and other diseases associated with the pathological activity of Notch receptors or in cases where it is appropriate to modulate the DII4-Notch signaling pathway.

The fusion proteins (recombinant polypeptides), which are the object of this invention, can be obtained as follows.

Soluble recombinant polypeptides antagonizing DII4 are produced as a result of the expression of nucleotide sequences encoding them in eukaryotic cell lines, followed by purification of the synthesized recombinant proteins by affinity, ionexchange or hydrophobic chromatography, used separately or in various combinations with each other, as well as by other methods of protein purification. The appropriate nucleotide sequences are obtained by combining the DNA regions encoding the selected DII4 domains with the DNA sequence encoding the Fc region of human IgG4. In some particular embodiments of the invention, nucleic acid molecules encoding such DII4 antagonist polypeptides, have a nucleotide sequence corresponding to SEQ ID NO: 3 or SEQ ID NO: 5.

This task is also solved by creating an expression vector containing this nucleic acid molecule under the control of regulatory elements necessary for the expression of this nucleic acid in the host cell. In the preferred embodiments of the invention, Chinese hamster ovary (CHO) cells (for example, CHO-K1 or CHO DG44 cell lines) adapted for the production of therapeutic proteins may act as a host cell that includes such an expression vector. In this invention, the expression vector is preferably selected for the expression of heterogeneous sequences in mammalian cells, but in some embodiments of the invention, the expression vector may be selected for expression in other systems, such as, for example, insect cells, yeast or bacterial cells. Accordingly, each expression vector has its own set of regulatory elements that allow the expression of a heterogeneous sequence (product) in the host cell, such as promoters and/or enhancers, Kozak sequences, polyA sequences, and other regulatory sequences. It can also have sequences encoding leader (signal) peptides, ensuring the secretion of recombinant polypeptides into the extracellular environment. The termination of protein synthesis from a given isolated nucleic acid sequence is determined by the addition of one or more stop codons to it at the 3'end in one reading frame.

After transfection of the vector into the cells of the eukaryotic cell line, the recombinant polypeptide is synthesized and secreted into the serum-free culture medium. The resulting recombinant polypeptide is isolated from the medium using, in most cases, protein A or protein G affinity chromatography; however, other protein purification methods can be used.

The unity of this invention is achieved through the use of a common minimum sequence of DII4 that interacts with Notch receptors in all constructs. This sequence includes amino acids 21-254 in SEQ ID NO: 2 and in SEQ ID NO: 4, and corresponds to the sequence SEQ ID NO: 6 and amino acids 171-404 in SEQ ID NO: 1. It includes DSL and the EGF(1-5)-like repeat domains of the human DII4 protein, which are necessary and sufficient for the interaction with Notch1 and Notch4 receptors. They do not contain the MNNL domain of DII4, which is involved in activating the Notch signal transmission.

In this invention, the DII4 antagonist contains amino acids 171-404 of the DII4 protein. This region does not include the 27-172 region corresponding to the MNNL domain (unlike, among other things, the polypeptides known from the patent EP2054082B1); as a result, the molecules of the invention cannot lead to the activation of Notch receptors. In addition, the deletion of the MNNL domain reduces aggregation of the protein and improves its solubility, which leads to an increase in protein production compared to fusion polypeptides with the full-size extracellular DII4 domain. Thus, this invention is unique and represents a new type of molecule that has not been described previously.

The essence of the technical solution lies in the creation of nucleotide sequences (in particular embodiments, SEQ ID NO: 3 and SEQ ID NO: 5) that encode the corresponding hybrid proteins with sequences (in particular embodiments, respectively, SEQ ID NO: 2 and SEQ ID NO: 4) that include two main functional parts. The first functional part corresponds to a part of the extracellular domain of human DII4, and in the presented particular embodiments of the invention corresponds to amino acids 21-254 for SEQ ID NO: 2 and amino acids 21-365 for SEQ ID NO: 4. The general DII4 sequence that combines these constructs corresponds to the sequence SEQ ID NO: 6. The second functional part encodes the constant part of the heavy chain of human IgG4 (SEQ ID NO: 7) and, in the presented particular embodiments of the invention, corresponds to amino acids 257-482 for SEQ ID NO: 2 and amino acids 368-593 for SEQ ID NO: 4.

The coding nucleotide sequences (including SEQ ID NO: 3 and SEQ ID NO: 5) can be changed to optimize the expression level in certain cell types. When heterogeneous expression systems (e.g., insect cells, bacterial or yeast cells) are used, it may be necessary to optimize codons in the sequence (substitution of codons rarely used in the body for commonly used ones). This can be done using algorithms implemented in many existing algorithms for designing sequences, such as Codon optimizer, Gene Designer, or OPTIMIZER. For industrial production of fusion proteins, the sequences corresponding to SEQ ID NO: 3 and SEQ ID NO: 5 can be recloned into any other vector, allowing the production and secretion of target polypeptides into the culture fluid, for subsequent purification of the products. In particular, the Freedom pCHO 1.0 vector (ThermoFisher) may be selected as such a vector.

Expression of the hybrid construct in mammalian cells may be achieved by creating stable producer clones after transfection of the cells with this construct. Transfection by electroporation or using a transfecting reagent, such as Lipofectamine 2000, may be used. This hybrid construct may also be used for introduction into a lentivirus construct and subsequent infection of cells. Different approaches may be used to increase the yield of the hybrid protein in mammalian cells. Optimization methods are known to those skilled in the art and are described, for example, in the publication (Almo et al. Better and faster: improvements and optimization for mammalian recombinant protein production // Curr Opin Struct Biol. 2014 Jun; 26:39-43).

The proposed products are a previously unknown combination of nucleotide sequences of the human genome (which have a common sequence corresponding to the Fc region of human IgG4 fused in the same reading frame with the fragment(s) of the human DII4 sequence) and are intended to serve as the expression basis for the production of the fusion hDII4-hFc protein, which has a whole complex of improved pharmacological properties, specifically: (1) minimal effector function of antibody-dependent cellular cytotoxicity (ADCC) and complement-dependent cytotoxicity (CDC) due to the use of the constant part of the human immunoglobulin IgG4 isotype; (2) a relatively small molecular weight, which increases the production efficiency of these molecules in mammalian cells and improves their properties related to the rate and extent of penetration into tissues; (3) the ability to effectively block Notch signaling due to the presence of functional domains necessary for effective binding to Notch receptors; (4) the inability to induce the activation of Notch receptors (due to the absence of the MNNL domain in the construct), as well as (5) the decreased aggregation ability of the protein and its improved solubility. After passing animal safety tests and clinical trials, the fusion protein of this invention can be included in a pharmaceutical composition for the treatment of diseases of the cardiovascular system, ischemic conditions, vascular inflammation, and cancers, for stimulating therapeutic angiogenesis.

The following examples are intended to disclose the characteristics of this invention and should not be regarded as limiting the scope of the invention in any way.

### Example 1. Plasmid construction

To construct plasmids with the claimed nucleotide sequences, the sequence encoding the human DII4 protein, obtained from the RG212628 plasmid (OriGene, DII4 (NM_019074) Human Tagged ORF clone), was used.

Fragments of DII4 were selected on the basis of bioinformatic sequence analysis, and the corresponding nucleotide sequences encoding them were used for subsequent cloning. For cloning the regions corresponding to amino acids 21-254 for SEQ ID NO: 2 and amino acids 21-365 for SEQ ID NO: 4, the following primers were selected:
*DLL4DMF:* AAGAATTCCTACCGGGTCATCTGCAGTGACAACTA (SEQ ID NO: 8)
*DLL4DMDE5R:* TAGGATCCGTCCACTTTCTTCTCGCAGTTGGA (SEQ ID NO: 9)
*DLL4DMR2:* TAGGATCCGCTGCCCACAAAGCCATAAGGGCA (SEQ ID NO: 10).

The primers included restriction sites for the restrictases EcoRI (direct primer DLL4DMF) and BamHI (reverse primers DLL4DMDE5R and DLL4DMR2).

A PCR was performed under the following conditions: at 95 °C for 1 min, 30 cycles (95 °C for 10 sec, 63 °C for 10 sec, 72 °C for 2 min), 72 °C for 5 min. Reaction components: ExTaq Takara polymerase (Takara Bio), buffer containing Mg2+ for ExTaq polymerase (Takara Bio), 12 pmol of each of the primers, and 0.25 mM of a mixture of nucleotide triphosphates (Takara Bio). After the PCR, the PCR product was purified with the PCR purification kit QIAquick (QiaGene Cat. No. 28106). The concentration and the correspondence of the molecular weight to the predicted one were checked using gel electrophoresis in 1% agarose (TAE buffer). The obtained PCR products were used for further construction.

The pFUSE-hIgG4-Fc2 vector (InvivoGen), which allows to obtain sequences fused with the Fc domain of IgG4 and examine their properties, was used to clone the target sequences. The vector was treated with the EcoRI and BgIII restrictases (Thermo), and the PCR product was treated with the EcoRI and BamHI restrictases (Thermo) for 1 hour at 37 °C; this was followed by purification using the PCR Purification kit QIAquick (Thermo) and ligation with 1 unit (IU) of ligase (Thermo) used according to the manufacturer's protocol. Competent E. *coli* XL10-Gold cells were transformed with the ligase mixture and seeded into LB medium containing Zeocin.

The dishes were incubated in a thermostat overnight at 37 °C. The next day, 20 colonies from each ligase mixture were tested for the presence of the desired insertion; to do this, part of the colony was diluted in 20 µL of water and boiled for 5 minutes. After cooling down, the mixture was centrifuged and 1 µL was used as a matrix in the PCR reaction. The presence of the insertion was checked after electrophoresis of the PCR products. The plasmid DNA was isolated from two colonies containing the insertion and sequenced on an Applied Biosystems 3500 sequencer according to the manufacturer's instructions, using the primers PROMF2 (direct) and FC (reverse).
*PROMF2:* GCCTGACCCTGCTTGCTCAACT (SEQ ID NO: 11)
FC: CTCACGTCCACCACCACGCA (SEQ ID NO: 12)

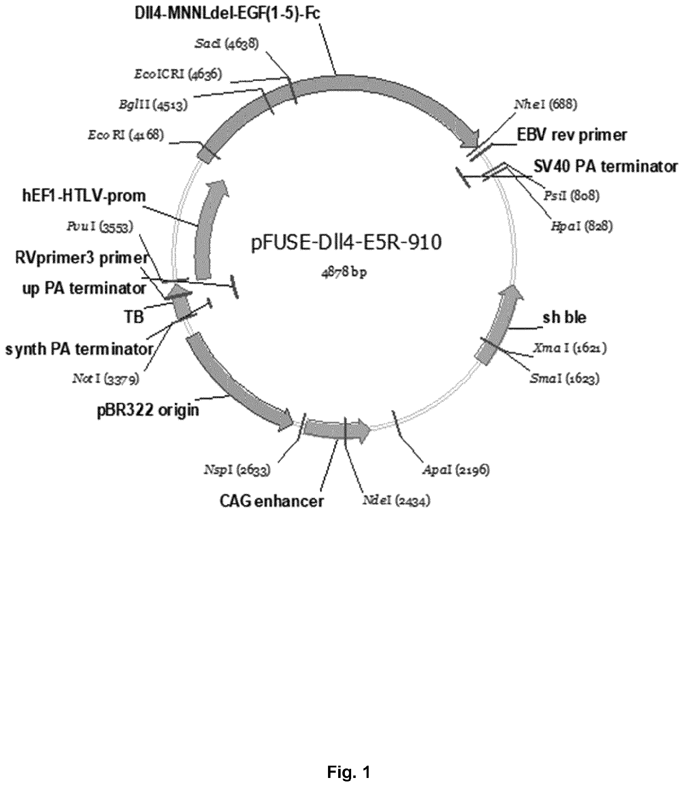

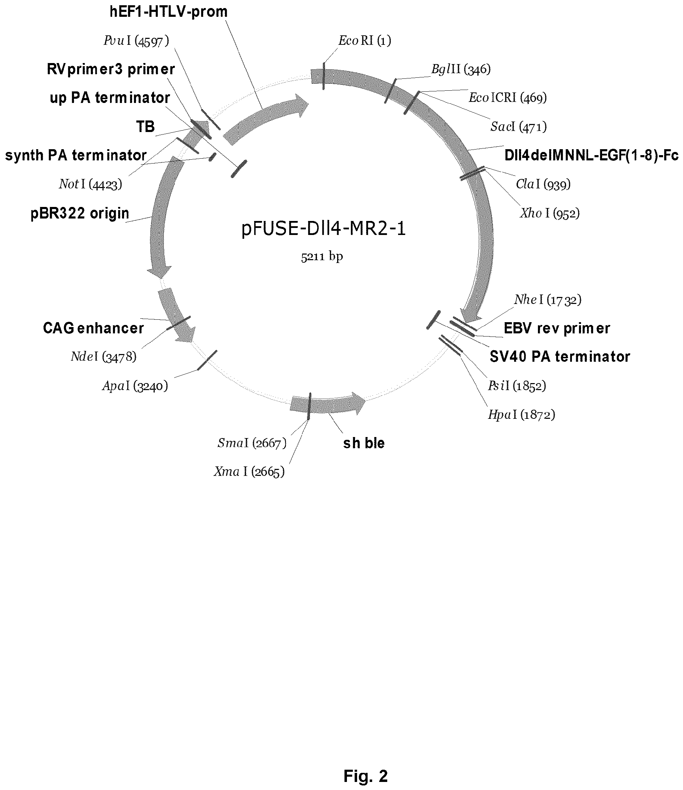

The constructed plasmids containing the target nucleotide sequences are shown in Figures 1 and 2.

### Example 2. Production of recombinant polypeptides

To transfect CHO cells, particularly pure ("transfer grade") plasmid DNA was isolated using the EndoFree Plasmid MaxiKit (Qiagen) according to the manufacturer's protocol. Both constructs were linearized at the Not1 site (Thermo). For the transfection, 20 µg of the linearized vector was added to 100 µL of a cell suspension with a concentration of 5×10⁷ cells/mL. Electroporation was performed according to the protocol: 3 impulses of 1130 V lasting 20 ms. Selection was started 48 hours after the transfection by adding the antibiotic Zeocin at a concentration of 500 µg/mL, and continued for further reseedings. Culture medium: Dynamis + 6 mM Glutamax + 0.5% anticlumping reagent B (Lonza, Switzerland) + 500 µg/mL Zeocin. Cultivation was carried out in a Multitron Cell CO₂ incubation shaker (Infors, Switzerland) in a 5% CO₂ atmosphere, at 37 °C and 95% relative humidity, in 125 mL Ernlenmeyer flasks (Corning, USA) (agitation at 120 rpm). The concentration of target polypeptides in the culture fluid was determined by bilayer interferometry using Protein A biosensors and the Octet K2 system (Fortebio, Pall, USA) in real time mode. The binding of the Fc region of the fusion proteins to Protein A immobilized on the sensor was evaluated. During the cultivation of these pools in the fed-batch mode, the production of the fusion polypeptides and their secretion into the culture fluid were confirmed. The level of production of the fusion proteins corresponding to the sequences SEQ ID NO: 2 and SEQ ID NO: 4 was significantly higher than the production level of the hybrid hDII4-hFc protein containing the full-size extracellular domain of DII4. The highest level of production was achieved for the molecule corresponding to SEQ ID NO: 2, which confirms the conclusion that the small size of the molecule and the absence of the MNNL domain play a fundamental role for the production of the protein in CHO cells. This fact is of critical importance for the industrial production of the protein, since effective production will decrease the cost of manufacturing a drug substance.

### Example 3. Assessment of the functional activity of the obtained recombinant polypeptides

Standard methods and approaches are used to evaluate the functional activity of the obtained recombinant hDII4-hFc polypeptides:
(1) comparative analysis of the binding of the recombinant hDII4-hFc polypeptide to the Notch1 and Notch4 target receptors using protein co-immunoprecipitation;
(2) study of the binding kinetics of the recombinant hDII4-hFc polypeptide to the Notch1 and Notch4 target receptors by analyzing the change in the angle of total internal reflection due to the surface plasmon resonance effect;
(3) study of the efficacy of suppression of Notch signaling in the cells by adding the recombinant hDII4-hFc polypeptide using real-time PCR, in particular, the change in the expression of the *HEY1, HEY2, HES1,* and *HES5* target genes.
(4) study of the angiogenic activity of the recombinant hDII4-hFc polypeptide in a HUVEC (human umbilical vein endothelial cells) model.

The procedure of the experiments is detailed below:
(1) According to the results of the literature analysis, the most likely natural targets of the obtained recombinant hDII4-hFc polypeptide are the proteins Notch1 and Notch4. As part of the study of the biological properties of hDII4-hFc, recombinant human proteins Notch1 and Notch4 obtained in a HEK293 cell suspension culture and purified by high-performance liquid chromatography (HPLC) are used as targets. At the first stage of the analysis, they are conjugated with biotin using the activated ester method, with subsequent purification on desalting columns. The hDII4-hFc polypeptide is produced in a suspension culture of CHO cells and purified using Protein A Sepharose liquid chromatography. After that, the test polypeptide is added to the biotinylated target proteins in a molar ratio of 1:1 in phosphate buffered saline (PBS, 1x, pH=7.4) with the addition of BSA, and the mixture is incubated at room temperature. After that, the polypeptide-target complexes are incubated with Dynabeads M280 magnetic beads (Invitrogen, USA) with streptavidin immobilized on their surface, washed, and eluted in accordance with the manufacturer's protocol. After that, the obtained complexes are purified, and the bound hDII4-hFc is detected using Western blotting.
(2) To evaluate the kinetics of the binding of the resulting hDII4-hFc polypeptide to Notch1 and Notch4, the surface plasmon resonance method in the BioRad Proteon XPR36 system is used. To do this, the previously obtained biotinylated recombinant proteins Notch1 and Notch4 are immobilized on a streptavidin-coated chip in HBS-EP buffer (pH=7.4). After that, the HBS-EP buffer containing varying concentrations of hDII4-hFc is passed through the analytical cell. When the solution is passed, the hDII4-hFc polypeptide is associated with target proteins. After that, the dissociation of the polypeptide and the target is evaluated. A free biotin solution is used to block unoccupied streptavidin sites in cells with Notch1, Notch4, as well as in the control. Data analysis is carried out in accordance with the manufacturer's protocol.
(3) The expression of the *HEY1, HEY2, HES1,* and *HES5* genes is performed on a primary HUVEC line model. For this purpose, the hDII4-hFc polypeptide purified as described in procedure (1) is added to the suspension of the HUVEC cells and incubated at 37 °C in a CO₂ incubator. HUVEC cells without added protein (buffer solution) are used as a control. After that, the cells are precipitated in a centrifuge, and total RNA is extracted from them. The concentration of the resulting RNA is measured using a Qubit 2.0 fluorimeter (Invitrogen, USA). The DNA is removed using the TurboDNA free kit (Invitrogen). After that, the reverse transcription reaction is performed, for which the same amount of RNA is taken from the different samples (test and control). Superscript III (Invitrogen) and random hexamers are used as primers for the reverse transcription. After that, the real-time PCR is performed on a CFX96 Bio-Rad amplifier using the Sso Fast EvaGreen supermix (Bio-Rad). The primer sequences for the amplification are presented below:
   *HES1 -* F: AAGAAAGATAGCTCGCGGCA (SEQ ID NO: 13)
   *HES1 -* R: TACTTCCCCAGCACACTTGG (SEQ ID NO: 14)
   *HES5 -* F: GATTCCTCTGTGTGGGTGGATG (SEQ ID NO: 15)
   *HES5 -* R: GATTTTATTATGGCGGCTTCGG (SEQ ID NO: 16)
   *HEY1* - F: CCTTCCCCTTCTCTTTCGGC (SEQ ID NO: 17)
   *HEY1 -* R: AAAAGCTCCGATCTCCGTCC (SEQ ID NO: 18)
   *HEY2 -* F: AAGGCGTCGGGATCGGATAA (SEQ ID NO: 19)
   *HEY2 -* R: AGAGCGTGTGCGTCAAAGTAG (SEQ ID NO: 20)
   *GAPDH* - F: CCATCACCATCTTCCAGGAG (SEQ ID NO: 21)
   *GAPDH -* R: AATGAGCCCCAGCCTTCTCC (SEQ ID NO: 22).
(4) The standard procedure, which is based on the cultivation of primary human umbilical vein endothelial cells (HUVEC) in a three-dimensional matrix, is used to assess the angiogenic activity of the resulting recombinant trap protein hDII4-hFc. To do this, primary endothelial cells are isolated from the umbilical vein of newborns, and then cultured in the M199 medium enriched with 20% fetal bovine serum and a cocktail of antibiotics (penicillin and streptomycin), in culture vials. When the cells achieve 70-80% confluence, the culture is passaged to the second passage according to the standard procedure. Two hours before transferring the cells into the three-dimensional matrix (Matrigel), they are transferred to a depleted EBM-2 medium containing 0.2% fetal bovine serum.

After that, the HUVEC cell suspension with a concentration of 5×10⁸ cells/mL is mixed with an equivalent volume of Matrigel. After that, 50 µL of the mixture is applied in the center of the well in 24-well plates. The test recombinant hDII4-hFc protein in varying concentrations is added to the cells in 1 mL of the EBM-2 medium one hour after the application and incubation at 37 °C. Each experiment is performed in three replicates. Cells without addition of the recombinant protein are used as controls. After that, the cells are incubated for 72 hours in a CO₂ incubator and fixed with 4% formalin in PBS.

The analysis of the formation of primary vessels (endothelial tubes) is carried out by the method of confocal microscopy. After fixing the Matrigel drops, they are permeated with a 0.5% solution of the Triton X-100 detergent. After that, the cells are stained with BDP 505/515 membrane dye and DAPI nuclear dye using a Leica TCS SP5 microscope (Leica, Germany). A software is used to characterize the primary vessels.

Recombinant hDII4-hFc polypeptides bind with a high selectivity to the Notch1 and Notch4 target receptors without activating them; as a result, such binding results in an effective blockade of Notch signaling, which is manifested, in particular, in the activation of the growth of new vessels (angiogenic activity) in ischemic tissues.

Although the invention has been described with reference to the disclosed embodiments, it will be apparent to those skilled in the art that the specific experiments described in detail are for illustrative purposes only and should not be construed as limiting the scope of the invention in any way. It should be appreciated that various modifications are possible without departing from the essence of the present invention.

## Claims

1. A DII4 antagonist fusion protein containing: a) a fragment of the soluble extracellular part of the human DII4 protein corresponding to the DSL domain and the domains of EGF-like repeats of the DII4 protein, wherein the DII4 protein fragment includes a DSL domain and at least domains 1-5 of EGF-like repeats, arranged in an order corresponding to the natural sequence; b) Fc-fragment of human lgG4 attached to the fragment of the protein DII4 (a) from the C-terminus directly or through a linker sequence.

2. The fusion protein according to claim 1, in which the DSL domain of the DII4 protein has an amino acid sequence corresponding to positions 173-217 of the sequence of SEQ ID NO: 1, and the domains of EGF-like repeats of the DII4 protein have amino acid sequences corresponding to positions 218-251, 252-282, 284-322, 324-360 and 362-400 sequences of SEQ ID NO: 1.

3. The fusion protein of claim 1, wherein the human IgG4 Fc fragment has the amino acid sequence of SEQ ID N0:7.

4. The fusion protein according to claim 1, additionally comprising at least one of the domains of 6-8 EGF-like repeats of the DII4 protein, located (s) from the C-terminus of the DII4 protein fragment (a), and the order of these domains is 6-8 EGF - such repetitions can be any.

5. The fusion protein of claim 4, wherein domains 6-8 of the EGF-like repeats of the DII4 protein have amino acid sequences corresponding to positions 402-438, 440-476 and 480-518 of SEQ ID N0:1.

6. The fusion protein of claim 1, wherein the human IgG4 Fc fragment (b) is linked to the DII4 protein fragment (a) in a linkerless manner.

7. The fusion protein of claim 1, wherein the human IgG4 Fc fragment (b) is connected to the DII4 protein fragment (a) via a linker peptide.

8. A fusion protein according to claim 4, wherein the human IgG4 Fc fragment (b) is linked to the DII4 protein fragment (a) via a GS linker peptide.

9. A fusion protein according to claim 1, wherein the DII4 protein fragment has the amino acid sequence of SEQ ID NO: 6.

10. The fusion protein of claim 1, further comprising a signal sequence located at the N-terminus of the fusion protein.

11. The fusion protein according to claim 1, having the amino acid sequence of SEQ ID NO: 2.

12. A fusion protein according to claim 4, having the amino acid sequence of SEQ ID NO: 4.

13. An isolated nucleic acid molecule encoding a fusion protein according to any one of claims 1-12.

14. An isolated nucleic acid molecule according to claim 13, having the sequence of SEQ ID NO: 3.

15. An isolated nucleic acid molecule according to claim 13, having the sequence of SEQ ID NO: 5.

16. An expression vector carrying a nucleotide sequence corresponding to the sequence of an isolated nucleic acid molecule according to any one of paragraphs 7-9 under the control of regulatory elements necessary for the expression of said nucleotide sequence in the host cell.

17. A cell capable of expressing a fusion protein according to any one of claims 1 to 12, which is not a human embryonic cell.

18. The cell according to claim 17, which is a Chinese hamster ovary (CHO) cell.
